# EUROPEAN PATENT APPLICATION

(11) **EP 3 943 077 A1**
(43) Date of publication of application: **26.01.2022**
(21) Application number: 20382660.7
(22) Date of filing: 22.07.2020
(51) Int. Cl.: A61K 31/047, A61K 36/14, A61P 3/00, A61P 25/00, A61P 27/02

(54) **USE OF JUNIPER (JUNIPERUS COMMUNIS) BERRIES EXTRACT AND AGATHADIOL AS POSITIVE ALLOSTERIC MODULATORS OF CANNABINOID TYPE 1 RECEPTOR**

(71) Applicant: Emerald Health Biotechnology España S.L., 14014 Córdoba (ES)
(72) Inventor: MUÑOZ BLANCO, Eduardo, 14004 Córdoba (ES); UNCITI BROCETA, Juan Diego, 14003 Córdoba (ES); BATTISTA APPENDINO, Giovanni, 10131 Torino (IT); POLLASTRO, Federica, 28070 Garbagna Novarese. Novara (ES)
(74) Representative: Manuel Illescas y Asociados, S.L.

(57) **Abstract**

It is disclosed a *Juniperus communis* extract comprising agathadiol, and pharmaceutical compositions comprising said extract, for use in the prevention and/or treatment of a disease or condition responsive to positive allosteric modulators (PAM) of cannabinoid type 1 receptor (CB 1R). Additionally, disclosed is agathadiol, as well as pharmaceutical compositions comprising the same, for use in the prevention and/or treatment of a disease or condition responsive to positive allosteric modulators cannabinoid type 1 receptor. Finally, also disclosed are nutraceutical compositions comprising agathadiol, or a *Juniperus communis* extract comprising the same, for preventing or alleviating the symptoms of a disease or condition responsive to positive allosteric modulators cannabinoid type 1 receptor.

## Description

### Field of the invention

The present invention relates to a *Juniperus communis* extract comprising agathadiol and to pharmaceutical compositions comprising said extract, for use in the prevention and/or treatment of a disease or condition responsive to positive allosteric modulators (PAM) of cannabinoid type 1 receptor (CB1R). Additionally, present invention also relates to agathadiol, as well as to pharmaceutical compositions comprising the same, for use in the prevention and/or treatment of a disease or condition responsive to PAM of CB1R. Finally, present invention also refers to a nutraceutical composition comprising agathadiol, or a *Juniperus communis* extract comprising agathadiol, for use in alleviating the symptoms of a disease or condition responsive to PAM of CB1R.

### Background of the invention

The proteins of the endocannabinoid system comprise at least two G-protein-coupled receptors (GPCRs), the cannabinoid type-1 receptor (CB1R) and the cannabinoid type-2 receptor (CB2R), as well as the enzymes for the synthesis and degradation of its endogenous agonists (anandamide, 2AG). The CB 1 receptor is the most abundant GPCR in the brain, with particularly high levels observed in the neocortex, hippocampus, basal ganglia, cerebellum, and brainstem. The CB1 receptor plays important roles in a variety of physiological conditions, including neuronal development, neuronal plasticity, food intake and energy balance, perception processes, immunomodulation, cell apoptosis, and cardiovascular and reproductive functions. This receptor it is widely distributed at lower concentrations in a variety of peripheral tissues (liver, adipose tissue, gastrointestinal tract, pancreas, adrenal grand, heart, lung, prostate, uterus, ovary, testis, bone marrow, thymus, tonsils and retina). In contrast, the CB2 receptor is primarily located in the periphery, with high concentrations in the tonsil, spleen, and immune-related cells.

The CB 1 receptor plays a critical neuromodulatory role in the central nervous system and regulatory role in peripheral tissues such as adipose and liver. CB 1R is considered a potential therapeutic target for the treatment of multiple sclerosis, Parkinson's disease, Huntington's disease, epilepsy, pain and appetite dysregulation and obesity. Despite exhaustive research, few cannabis-based therapeutics have reached clinical use, although nabilone (Cesamet), dronabinol (Marinol) and a Δ9-tetrahydrocannabinol (THC)/cannabidiol (CBD) blend (Sativex) are approved for the treatment of spasticity, nausea and pain in various countries.

Conventional drug design has primarily targeted the orthosteric site of cannabinoid receptors, by means of designing and creating ligands that compete with the endogenous cannabinoid ligands (endogenous CB1R orthosteric ligands or endocannabinoids), anandamide (N-arachidonoylethanolamide) and 2-arachidonoyl-glycerol (2-AG), for binding to this site. However, psychoactive side effects are frequent and often preclude clinical usefulness for ligands that target these sites on the CB1R. Thus, research into the design and development of CB1R-targeted therapeutics has yet to yield clinically efficacious and safe drugs.

Historically, drug discovery programs aimed at regulating GPCR functions have been dominated by the identification of ligands to compete with endogenous ligands at the orthosteric sites. Recently, tremendous advances have been achieved in the discovery of ligands that regulate GPCR functions by binding to receptor sites that are topographically distinct from orthosteric sites, defining these compounds as allosteric modulators. Allosteric ligand modulators of GPCRs induce and stabilize unique conformations of GPCRs and therefore provide fundamentally different receptors that are capable of exerting novel pharmacological effects.

The binding of the allosteric ligands may also modulate the binding affinity of orthosteric ligands, the signaling efficiency of orthosteric ligands, or may perturb signaling even in the absence of orthosteric ligands.

There are four types of allosteric modulators; 1) Potentiators or positive allosteric modulators (PAMs) are ligands that increase receptor function. The positive modulation can be seen by an increase in agonist affinity or efficacy; 2) Allosteric antagonists or negative allosteric modulators (NAMs) are ligands that decrease receptor function through a decrease in agonist affinity or efficacy; 3) Allosteric agonists (ago-allosterics or ago-agonists) are allosteric compounds that display positive modulation in the absence of the orthosteric ligand; 4) Neutral allosteric ligands (NALs) are ligands that bind at the allosteric site but do not modulate receptor function.

Positive allosteric modulators (PAMs) enhance, therefore, the receptor signalling. There are several scenarios of PAMs action: the receptor may have increased affinity to the orthosteric ligand or the orthosteric ligand may dissociate less efficiently from the receptor. Also, the increased potency or increased efficacy of the orthosteric ligand and some combination of the above may contribute to enhanced signaling.

Allosteric modulators have several advantages that make them potentially more effective than the orthosteric ligands.

The most important is the enhanced specificity due to a higher level of the sequence variability in allosteric binding sites compared to conservative orthosteric domains, enabling a specific action on a given receptor subtype. In fact, since most allosteric sites are not constrained by high evolutionary pressure to maintain endogenous ligand binding, they may not have high sequence identity across species or between subtypes. Thus, targeting these sites could help developing subtype-specific drugs, but also emphasizes the challenges of identifying and characterizing allosteric modulators across species.

Additionally, allosteric sites offer an opportunity to develop therapeutics for receptors where targeting the orthosteric site has not yielded successful drugs.

Another important feature is that "pure" allosteric modulators may have an effect only when the endogenous/orthosteric ligand is present and thus, maintain the temporal and spatial characteristics of endogenous cell signalling. PAMs, for example, may amplify endogenous cell signalling without affecting its temporal regulation. This could be of great importance especially with neurotransmitters where signal timing is crucial.

Moreover, orthosteric ligands may cause significant desensitization/downregulation of receptors due to constant receptor activation. Since allosteric activity may not activate cellular pathways leading to desensitization and down regulation, in some instances these allosteric modulators offer potential for lesser receptor desensitization and/or behavioural tolerance.

Other advantages of the allosteric modulators are the *saturability* or ceiling effect (no further modulation is observed beyond a certain concentration of the allosteric ligand, protecting from overdosing) as well as probe dependence (the same allosteric ligand may have different effects on different orthosteric ligands). Accordingly, allosteric modulators can be used to develop ligands with better adverse effect profiles than efficacious orthosteric ligands, where increasing concentrations may lead to undesirable adverse events.

For those reasons, the development of CB1R allosteric modulators has become an area of immense importance within the cannabinoid field. It is clear that enormous potential exists to successfully target CB1R through allosteric sites for the treatment of different types of pain, inflammation, neurological disorders including neurodegenerative diseases, movement disorders (e.g. Huntington's disease), psychiatric, glaucoma, addiction, metabolic disorders such as obesity, and other pathological conditions where other avenues - such as agonists and inverse agonists - have been unsuccessful.

### Brief description of the invention

Present invention refers to a plant extract comprising agathadiol, or to a pharmaceutical composition comprising said plant extract, for use in the prevention and/or treatment of a disease or condition responsive to positive allosteric modulators (PAM) of cannabinoid type 1 receptor (CB1R).

Another aspect of present invention refers to agathadiol for use in the prevention and/or treatment of a disease or condition responsive to PAM of CB1R.
Yet another aspect of the present invention also refers to a pharmaceutical composition comprising agathadiol, and at least one pharmaceutically acceptable excipient, for use in the prevention and/or treatment of a disease or condition responsive to PAM of CB1R.

Finally, another aspect of present invention refers to a nutraceutical composition comprising agathadiol, or a plant extract which comprises agathadiol, for use in alleviating the symptoms of a disease or condition responsive to PAM of CB1R.

### Brief description of the figures

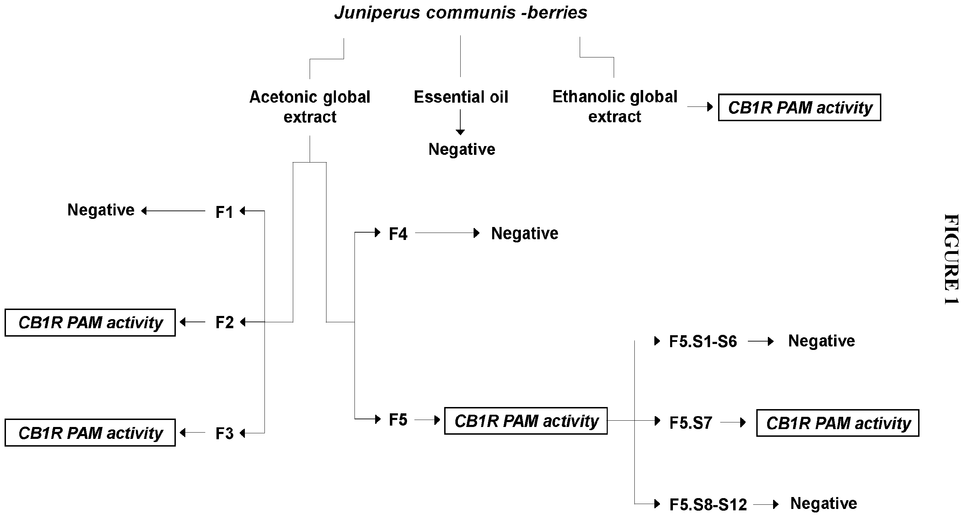

**Figure 1****. Schematic illustration of CB1R positive allosteric modulation activity of Juniper fractions obtained by solvent extraction methods:** The scheme indicates CB1R PAM activity of Juniper fractions obtained by different solvent extraction-fractionation methods (Acetonic extract, essential oil and ethanolic extract). F1, F2 and F3 correspond, respectively, to the petroleum ether, ethyl acetate and tetrahydrofuran fractions obtained by an increasing polarity (petroleum ether, ethyl acetate, tetrahydrofuran) solid phase extraction method from a global initial extract prepared by extraction with acetone. F4 and F5 correspond, respectively, to the acidic and neutral phases obtained by extraction liquid/liquid with 2% NaOH from a global initial extract prepared by extraction with acetone. F5.S1-7 fractions correspond to neutral phase (F5) purified on silica gel. F5.S8-12 correspond to neutral phase (F5) purified on silica gel and filtered on alumina. **CB1R PAM activity** means that the fractions or compound tested increases the activity induced by the CB1R orthosteric ligand CP-55,940, which is considered 100% activation. Values about 150% of activation are considered PAM and fractions and compounds with values of 100% in the presence of CP-55,940 are considered negative as PAM.

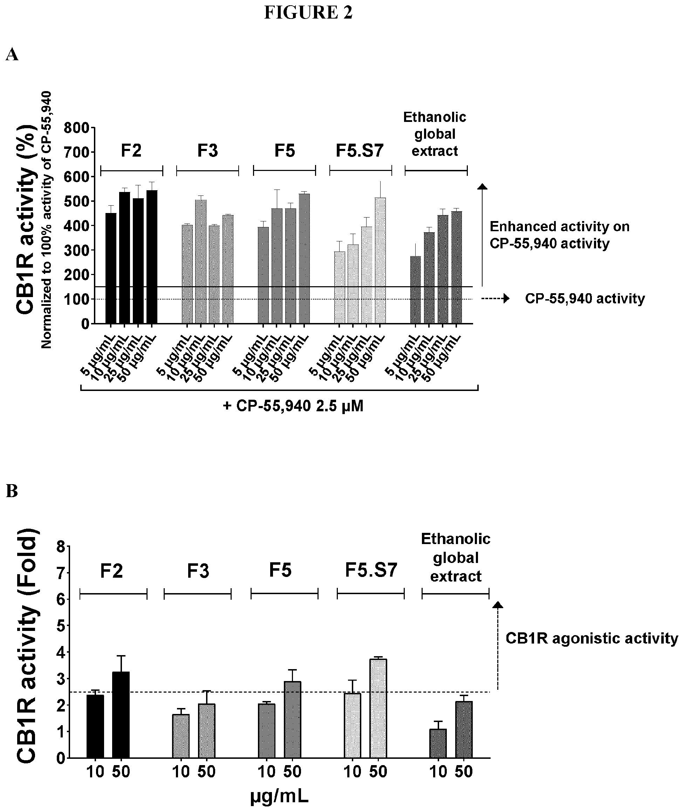

**Figure 2****. Effect on CB1R activity of Juniper fractions obtained by solvent extraction methods.** (A) CB1R activity of F2, F3, F5 and F5.S7 fractions, and ethanolic global extract at different concentrations (5, 10, 25 and 50 µg/mL) in the presence of 2.5 µM CP-55,940 on HEK293T-CB1-CRE-luc cells. Results are expressed as percentage of CB1R activity normalized to 100% stimulation induced by 2.5 µM CP-55,940 alone (bound to the orthosteric site). Bars represent the mean (% CB1R activity) ± SEM from 3 independent experiments performed in duplicate. CB1R activity ≥ 150% is considered CB1R PAM activity. **(B)** CB1R activity of F2, F3, F5 and F5.S7 fractions, and ethanolic global extract at different concentrations (10 and 50 µg/mL) on HEK293T-CB1-CRE-luc cells. Results are expressed as fold change of CB1R activity over non-stimulated cells. Bars represent the mean (fold change of CB1R activity) ± SEM from 2 independent experiments performed in duplicate. CB 1R activity ≥ 2.5-fold increase is considered CB1R agonistic activity. **F2:** Ethyl acetate fraction. F3: methanol fraction. F5: Neutral fraction. F5.S7: Neutral phase (F5) purified on silica gel and filtered on alumina.

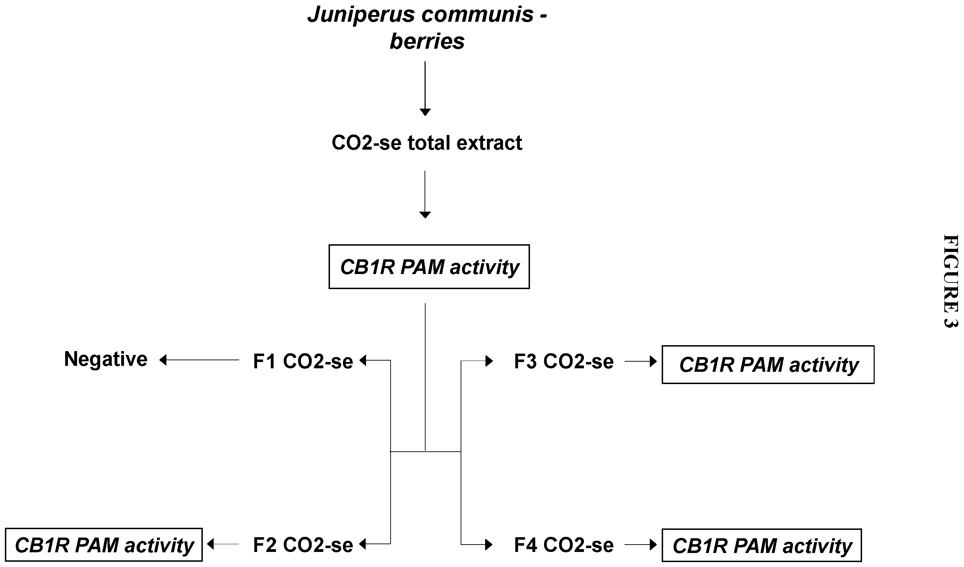

**Figure 3****. Schematic illustration of CB1R positive allosteric modulation activity of Juniper CO2-se total extract and derived fractions:** The scheme indicates CB1R PAM activity of Juniper CO2-se total extract and fractions obtained by a liquid/liquid (petroleum ether:methanol/water 9:1 v/v) partition method from the Juniper CO2-se total extract. F1 CO2-se and F2 CO2-se fractions correspond, respectively, to petroleum ether fraction and to petroleum ether fraction filtered on alumina. F3 CO2-se and F4 CO2-se fractions correspond, respectively, to methanol/water fraction and to methanol/water fraction filtered on alumina. **Juniper CO2-se total extract:** Juniper supercritical carbon dioxide extract. **CB1R PAM activity** means that the fractions or compound tested increases the activity induced by the CB1R orthosteric ligand CP-55,940, which is considered 100% activation. Values about 150% of activation are considered PAM and fractions and compounds with values of 100% in the presence of CP-55,940 are considered negative as PAM.

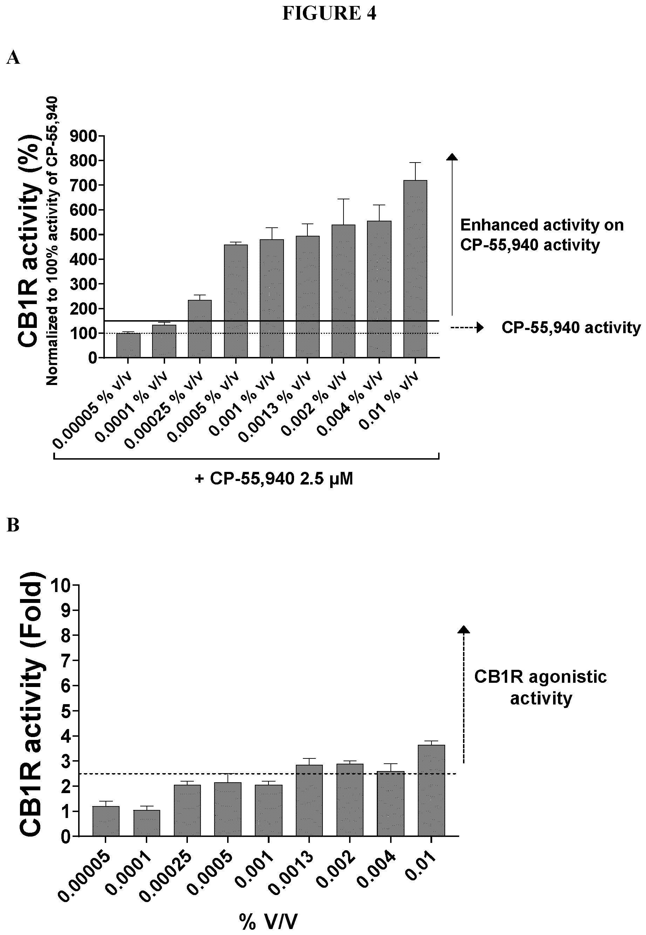

**Figure 4****. Effect on CB1R activity of Juniper CO2-se total extract. (A)** CB1R activity of Juniper CO2-se extract at different concentrations (0.00005; 0.0001; 0.00025; 0.0005; 0.001; 0.0013; 0.002; 0.004 and 0.01 % V/V) in the presence of 2.5 µM CP-55,940 on HEK293T-CB1-CRE-luc cells. Results are expressed as percentage of CB1R activity normalized to 100% stimulation induced by 2.5 µM CP-55,940 alone (bound to the orthosteric site). Bars represent the mean (% CB1R activity) ± SEM from 3 independent experiments performed in duplicate. CB1R activity ≥ 150% is considered CB1R PAM activity. **(B)** CB1R activity of Juniper CO2-se extract at different concentrations (0.00005; 0.0001; 0.00025; 0.0005; 0.001; 0.0013; 0.002; 0.004 and 0.01 % V/V) on HEK293T-CB1-CRE-luc cells. Results are expressed as fold change of CB1R activity over non-stimulated cells. Bars represent the mean (fold change of CB1R activity) ± SEM from 2 independent experiments performed in duplicate. CB1R activity ≥ 2.5-fold increase is considered CB1R orthosteric agonistic activity.

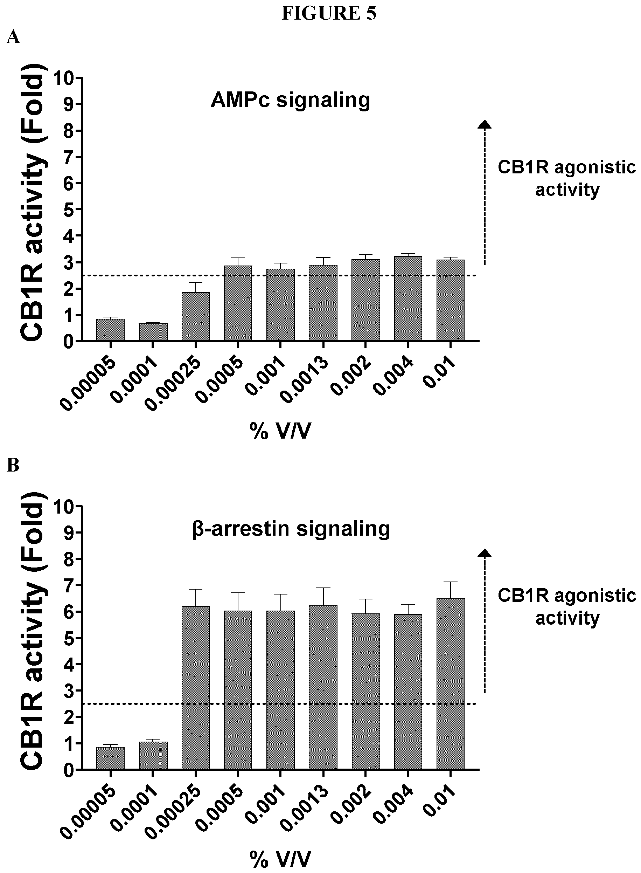

**Figure 5****. Effect on CB1R-mediated signalling pathways of Juniper CO2-se total extract.** Activity on CB1R-mediated signalling pathways (cAMP **-graph A-** and β-arrestin **-graph B-)** of Juniper CO2-se extract at different concentrations (0.00005; 0.0001, 0.00025, 0.0005, 0.001, 0.0013, 0.002, 0.004 and 0.01 % v/v) on Nomad CB1 cells. Results are expressed as fold change of CB1R activity over non-stimulated cells. Bars represent the mean (fold change of CB1R activity) ± SEM from 2 independent experiments performed in duplicate. Activity ≥ 2.5-fold is considered CB1R agonistic activity for the specific signalling pathway.

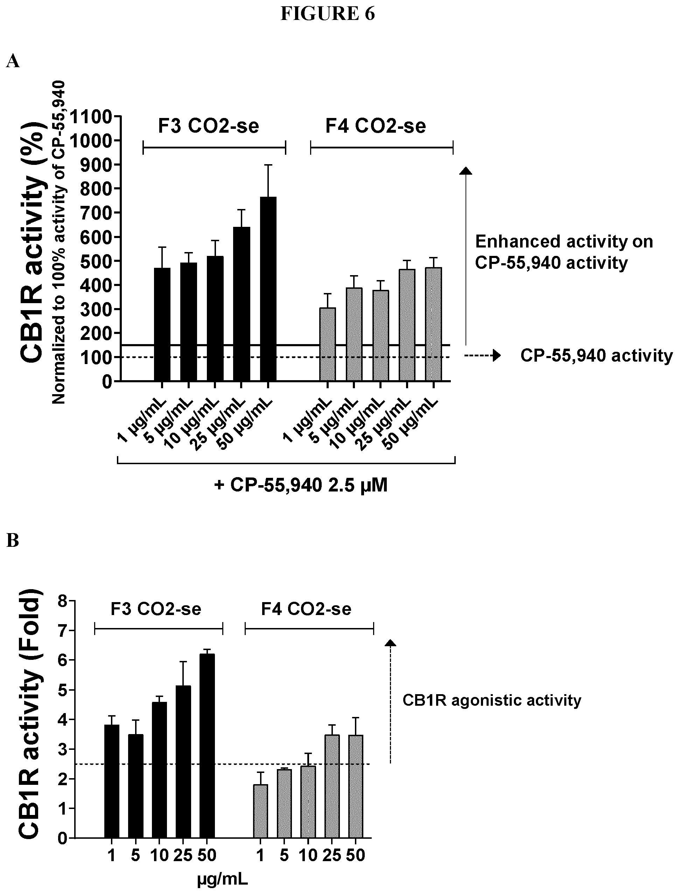

**Figure 6****. Effect on CB1R activity of Juniper methanol/water fractions obtained from CO2-se total extract.** (A) CB1R activity of Juniper fractions F3 CO2-se and F4 CO2-se at different concentrations (1, 5, 10, 25 and 50 µg/mL) in the presence of 2.5 µM CP-55,940 on HEK293T-CB1-CRE-luc cells. Results are expressed as percentage of CB1R activity normalized to 100% stimulation induced by 2.5 µM CP-55,940 alone (bound to the orthosteric site). Bars represent the mean (% CB1R activity) ± SEM from 3 independent experiments performed in duplicate. CB1R activity ≥ 150% is considered CB1R PAM activity. **(B)** CB1R activity of Juniper fractions F3 CO2-se and F4 CO2-se at different concentrations (1, 5, 10, 25 and 50 µg/mL) on HEK293T-CB1-CRE-luc cells. Results are expressed as fold change of CB1R activity over non-stimulated cells. Bars represent the mean (fold change of CB1R activity) ± SEM from 2 independent experiments performed in duplicate. CB1R activity ≥ 2.5-fold increase is considered CB1R agonistic activity. **F3 CO2-se:** methanol/water fraction. F4 **CO2-se:** methanol/water fraction filtered on alumina.

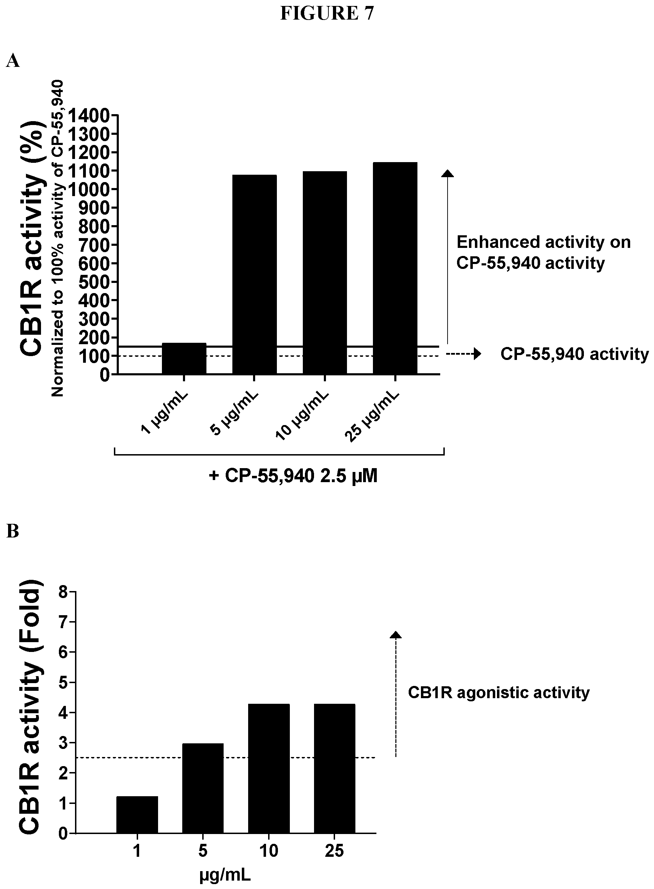

**Figure 7****. Effect on CB1 activity of agathadiol isolated from F4 CO2-se fraction. (A)** CB1R activity of agathadiol at different concentrations (1, 5, 10 and 25 µg/mL) in the presence of 2.5 µM CP-55,940 on HEK293T-CB1-CRE-luc cells. Results are expressed as percentage of CB1R activity normalized to 100% stimulation induced by 2.5 µM CP-55,940 alone (bound to the orthosteric site). Bars represent % CB1R activity. CB1R activity ≥ 150% is considered CB1R PAM activity. (B) CB1R activity of agathadiol at different concentrations (1, 5, 10 and 25 µg/mL) on HEK293T-CB1-CRE-luc cells. Results are expressed as fold change of CB1R activity over non-stimulated cells. Bars represent the fold change of CB1R activity. CB1R activity ≥ 2.5-fold increase is considered CB1R agonistic activity.

### Detailed description of the invention

Present invention refers to a plant extract comprising agathadiol [IUPAC name (E)-5-[(1S,4aR,5S,8aR)-5-(hydroxymethyl)-5,8a-dimethyl-2-methylidene-3,4,4a,6,7,8-hexahydro-1H-naphthalen-1-yl]-3-methylpent-2-en-1-ol, CAS number 1857-24-5] for use in the prevention and/or treatment of a disease or condition responsive to positive allosteric modulators (PAM) of cannabinoid type 1 receptor (CB1R).
For the purposes of the present invention the term "a disease or condition responsive to positive allosteric modulators of cannabinoid type 1 receptor", or "a disease or condition responsive to PAM of CB1R", refers to a disease or condition which responds to the treatment with positive allosteric modulators of cannabinoid type 1 receptor, i.e., a disease or condition whose treatment benefits from the administration of positive allosteric modulators of cannabinoid type 1 receptor, improving or alleviating the pathological state of the patient suffering from said disease or condition.

In an embodiment of present invention said disease or condition responsive to positive allosteric modulators of cannabinoid type 1 receptor (PAM of CB1R) is selected from the group consisting of neurological diseases, psychiatric diseases, glaucoma, inflammation, metabolic and eating disorders, pain, additive behaviour and substance-use disorders.

In another embodiment of the present invention, said disease or condition responsive to PAM of CB1R is selected from the group consisting of a neurological and psychiatric disorders associated with endocannabinoid dysfunction, glaucoma, inflammation, metabolic and eating disorders, pain, additive behaviour and substance-use disorders.

Preferably the neurological disorder is selected from the group consisting of neurodegenerative diseases and movement disorders. More preferably, said neurological disorder is selected from the group consisting of multiple sclerosis, Parkinson's disease, Huntington's disease, Alzheimer disease and epilepsy, among others.

Preferably, the psychiatric disorder is selected from the group consisting of psychotic disorders, anxiety, depression, bipolar disorder, panic disorder, obsessive-compulsive disorder, schizophrenia, post-traumatic stress disorder, personality disorders, among others.

Preferably, the inflammation or inflammatory disorders are selected from the group consisting of inflammatory bowel disorders, Crohn syndrome, arthrosis, arthritis, among others.

Preferably, metabolic and eating disorders are selected from the group consisting of metabolic syndrome, dyslipidemia, obesity, anorexia, appetite dysregulation, nausea, among others.

Preferably the pain is neuropathic pain or inflammatory pain. Neuropathic pain is caused by the somatosensory nervous system and does not feature inflammatory symptoms. Neuropathic pain may be selected from pain originated by cancer, chemotherapy (chemotherapy-induced peripheral neuropathy), radiation injury, surgery, spinal cord injury, multiple sclerosis, stroke, diabetes, metabolic disorders, herpes zoster, HIV, and immune disorders.

In a particularly preferred embodiment, the disease or condition responsive to PAM of CB1R is selected from the group consisting of multiple sclerosis, Parkinson's disease, Huntington's disease, Alzheimer disease, epilepsy, psychotic disorders, depression, bipolar disorder, panic disorder, obsessive-compulsive disorder, schizophrenia, post-traumatic stress disorder, personality disorders, glaucoma, inflammatory bowel disorders ,Crohn syndrome, arthrosis, arthritis, metabolic syndrome, dyslipidemia, obesity, anorexia, obesity, appetite dysregulation, nausea, neuropathic pain, inflammatory pain, and additive behaviour and substance-use disorders.

The genus *Juniperus* (family Cupressaceae) is an evergreen aromatic shrub or tree mostly distributed throughout the cold and temperate regions of Northern Hemisphere with some species extending as far South as Tropical Africa The berries have long been used as medicine by many cultures and are used in traditional medicine for diverse applications but without a common aetiology. Additionally, berries have been traditionally used as a spice due to its scent and flavour. On the other hand, it has been reported that the natural mixture in the *Juniperus communis* alcoholic extract composition could be used to treat cancer because it has preventive properties specific to the cell cycle that can be explained by the content in deoxypodophyllotoxin.

The phytochemical profile of *J. communis* is dominated by the production of isoprenoids, both volatiles and non-volatile. The volatile compounds are present in the essential oil, while extracts prepared with organic solvents (acetone, ethanol) or with supercritical CO₂ also contain the non-volatile isoprenoids. The diterpene alcohols agathadiol and the hydroxyaldehyde agathadial have been isolated as minor neutral diterpenoids from the berries. In any case, either agathadiol or deoxypodophyllotoxin are present in berries in a very low concentration, which could not justify the various medicinal uses accounted in traditional medicine for said berries.

Related Juniper species contain compounds belonging to the same phytochemical families than those obtained from *J. communis,* but significant differences exist within each class, so that, from a phytochemical standpoint, Juniper extracts can be distinguished on the basis of their non-overlapping phytochemical profile.

A first screening of various extracts of Juniper berries, included in Example 1 of present application, showed that the essential oil was inactive as a PAM of CB 1R, ruling out the involvement of volatile isoprenoids in the activity. On the other hand, all ethanol and acetone extracts (as shown in Example 1), as well as supercritical carbon dioxide extracts (as shown in Example 3), showed significant bioactivity. Since the carbon dioxide extracts were devoid of polar flavonoids and polyphenolics, it was determined that the active ingredients were compounds of intermediate polarity.

In order to identify the active fraction, the primary extracts (acetone, ethanol, carbon dioxide) were partitioned between an organic solvent and a basic water solution. In this way, the major acidic diterpenoid fraction could be separated from the extracts and recovered by acidification but turned out inactive.

Therefore, for the isolation of the active compounds an acetone extract was prepared. After removal of the acidic constituents by extraction with bases, the residue was fractionated by gravity column chromatography using a hexane-ethyl acetate gradient. Fractions were combined according to their TLC profile, and all fractions were assayed. Bioactivity was determined for two fractions, containing mainly, the diterpene alcohol agathadiol.

Accordingly, the *Juniperus communis* extract comprising agathadiol as well as the compound agathadiol *per se,* are useful as PAM of CB1R. More preferably, the *Juniperus communis* extract comprising agathadiol is a *Juniperus communis* berries extract and, more preferably a *Juniperus communis* berries supercritical CO₂ extract.

In an embodiment, the *Juniperus communis* extract comprising agathadiol, or the compound agathadiol per se, are obtained from berries of *Juniperus communis* and, in particular, from a *Juniperus communis* berries extract and, more preferably from a *Juniperus communis* berries supercritical CO₂ extract. In another embodiment of present invention agathadiol is obtained by a synthetic process, preferably departing from agathic acid [(1S,4aR,5S,8aR)-5-[(E)-4-carboxy-3-methylbut-3-enyl]-1,4a-dimethyl-6-methylidene-3,4,5,7,8,8a-hexahydro-2H-naphthalene-1-carboxylic acid, CAS number 640-28-8] or its monoesters.

For the purposes of present invention the term "monoester" in relation to agathic acid makes reference to the fact that only one of the two carboxylic acid groups of said compound have been esterified.

One preferred embodiment of present invention refers, accordingly, to a *Juniperus communis* extract comprising agathadiol for use in the prevention and/or treatment of a disease or condition responsive to PAM of CB1R.

For the purposes of present invention, the term "agathadiol" refers to (1S,4aR,5S,8aR)-5-(hydroxymethyl)-5,8a-dimethyl-2-methylidene-3,4,4a,6,7,8-hexahydro-1H-naphthalen-1-yl]-3-methylpent-2-en-1-ol, for instance, as obtained from the *Juniperus communis* berries extracts disclosed in present invention, but also referred to other enantiomers, diastereomers and E/Z isomers, or conformational isomers thereof.

An embodiment of the invention disclosed in present application refers to the use of a plant extract comprising agathadiol, in the manufacturing of a medicament for the prevention and/or treatment of a disease or condition responsive to PAM of CB1R. Preferably, present application refers to the use of a *Juniperus communis* extract comprising agathadiol, in the manufacturing of a medicament for the prevention and/or treatment of a disease or condition responsive to PAM of CB1R.

Another embodiment of present application refers to a method for preventing and/or treating a disease or condition responsive to PAM of CB1R in a subject in need thereof, wherein said method comprises administering an effective amount of a plant extract comprising agathadiol, and more preferably a *Juniperus communis* extract comprising agathadiol, to said subject.

For the purposes of present invention, an effective amount of the compound, when said effective amount is referred to a therapeutic use of said compound, is that which provides a therapeutic effect in the subject or an objectively identifiable improvement in the health condition of said subject as noted by the clinician or other qualified observer.

The dosage of compounds used in accordance with the invention, defining said dose the effective amount of said compounds, varies depending on the compound and the condition being treated for example the age, weight, and clinical condition of the recipient patient. Other factors include: the route of administration, the patient, the patient's medical history, the severity of the disease process, and the potency of the particular compound. The dose, or effective amount, should be sufficient to ameliorate symptoms or signs of the disease treated without producing unacceptable toxicity to the patient. Agathadiol provided and increasingly stronger bioactive effect as PAM of CB1R with increasing concentrations. Accordingly, from example 5 it can be concluded that agathadiol exerts clear CB1R PAM activities at a range of concentrations.

The term "comprises" indicates that includes a group of certain features (for example a group of features A, B and C) is interpreted as meaning that it includes those features (A, B and C), but that it does not exclude the presence of other features (for example features D or E), as long as they do not render the claim unworkable. Additionally, the terms "contains", "includes", "has" or "encompass", and the plural forms thereof, should be taken as synonymous of the term "comprises" for the purposes of present invention. On the other hand, if the wording "consists of' is used, then no further features are present in the apparatus/method/product apart from the ones following said wording. In this sense, for the purposes of present invention, the term "comprises" may be replaced by any of the terms "consists of', or "consists essentially of'. Accordingly, "comprises" may refer to a group of features A, B and C, which additionally may include other features, such as E and D, with the condition that said features do not render the claim unworkable, but said term "comprises" also includes the situation in which the group of features "consists of' or "consists essentially" of A, B and C.

An additional aspect of present invention refers, as well, to a pharmaceutical composition comprising a plant extract which comprises agathadiol, and at least one pharmaceutically acceptable excipient, for use in the prevention and/or treatment of a disease or condition responsive to PAM of CB1R. Preferably, said plant extract is a *Juniperus communis* extract comprising agathadiol, and one preferred embodiment of present invention refers, therefore, to a pharmaceutical composition comprising a *Juniperus communis* extract which comprises agathadiol and at least one pharmaceutically acceptable excipient, for use in the prevention and/or treatment of a disease or condition responsive to PAM of CB1R.

The excipient included in the pharmaceutical compositions for use according to present invention, refers, for the purpose of present invention, to an inert ingredient such as, but not limited to, cosolvents, surfactants, oils, humectants, emollients, preservatives, stabilizers and antioxidants. Any pharmacologically acceptable buffer may be used, such as TRIS or any phosphate buffer.

One embodiment also disclosed herein refers to the use of a pharmaceutical composition comprising a plant extract which comprises agathadiol; and at least one pharmaceutically acceptable excipient, in the manufacturing of a medicament for the prevention and/or treatment of a disease or condition responsive to PAM of CB1R. Preferably, one embodiment refers to the use of pharmaceutical composition comprising a *Juniperus communis* extract which comprises agathadiol, and at least one pharmaceutically acceptable excipient, in the manufacturing of a medicament for the prevention and/or treatment of a disease or condition responsive to PAM of CB1R.

Another embodiment disclosed herein refers to a method for preventing and/or treating a disease or condition responsive to PAM of CB1R in a subject in need thereof, wherein said method comprises administering an effective amount of a pharmaceutical composition comprising a plant extract which comprises agathadiol, more preferably a *Juniperus communis* extract which comprises agathadiol, and at least one pharmaceutically acceptable excipient to said subject.

Another aspect of present invention refers to agathadiol for use in the prevention and/or treatment of a disease or condition responsive to PAM of CB1R.

One embodiment also disclosed herein refers to the use of agathadiol in the manufacturing of a medicament for the prevention and/or treatment of a disease or condition responsive to PAM of CB1R.

Another embodiment of the invention disclosed herein refers to a method for preventing and/or treating a disease or condition responsive to PAM of CB1R in a subject in need thereof, wherein said method comprises administering an effective amount of agathadiol to said subject.

An additional aspect of the present invention refers to a pharmaceutical composition comprising agathadiol and at least one pharmaceutically acceptable excipient, for use in the prevention and/or treatment of a disease or condition responsive to PAM of CB1R.

One embodiment of the invention also disclosed herein refers to the use of a pharmaceutical composition comprising agathadiol and at least one pharmaceutically acceptable excipient, in the manufacturing of a medicament for the prevention and/or treatment of a disease or condition responsive to PAM of CB1R.

Another embodiment of the present invention disclosed herein refers to a method for preventing and/or treating a disease or condition responsive to PAM of CB1R in a subject in need thereof, wherein said method comprises administering an effective amount of a pharmaceutical composition comprising agathadiol and at least one pharmaceutically acceptable excipient, to said subject.

In one embodiment of the present invention disclosed in the present application, the pharmaceutical compositions disclosed herein may optionally comprise at least a second active compound having additive or synergistic biological activities.
For the purposes of present description, the term "active compound or active principle" should be taken as synonyms and mean a chemical entity which exerts therapeutic effects when administered to human or animal beings.

Additionally, the plant extract comprising agathadiol, more preferably the *Juniperus* extract comprising agathadiol, the compound agathadiol *per se,* and the pharmaceutical compositions comprised in present application may be administered in combination with, prior to, or after administering another active compound or principle, or in combination with, prior to, or after another therapy.

Preferably, the plant extract comprising agathadiol, more preferably the *Juniperus* extract comprising agathadiol, the compound agathadiol *per se,* and the pharmaceutical compositions comprised in present application may be administered in combination with, prior to, or after a medicament for treating neurological diseases, psychiatric diseases, glaucoma, inflammation, metabolic and eating disorders, pain, additive behaviour and substance-use disorders. Preferably, in combination with, prior to, or after a medicament for treating multiple sclerosis, Parkinson's disease, Huntington's disease, Alzheimer disease, epilepsy, psychotic disorders, depression, bipolar disorder, panic disorder, obsessive-compulsive disorder, schizophrenia, post-traumatic stress disorder, personality disorders, glaucoma, inflammatory bowel disorders ,Crohn syndrome, arthrosis, arthritis, metabolic syndrome, dyslipidemia, obesity, anorexia, obesity, appetite dysregulation, nausea, neuropathic pain, inflammatory pain, and additive behaviour and substance-use disorders.

The PAM of CB1R disclosed in present application (i.e. agathadiol) increases the effect provided by the endogenous orthosteric ligands of CB1R or endocannabinoids, anandamide and 2-arachidonoylglycerol and, therefore, the administration of the plant extract comprising agathadiol, more preferably the *Juniperus* extract comprising agathadiol, the compound agathadiol as such, and the pharmaceutical compositions of the invention comprising the same (both the extracts and agathadiol, as disclosed in present invention), increase the activity provided by said endocannabinoids.

Accordingly, in a more preferred embodiment of the invention, the plant extract comprising agathadiol, more preferably the *Juniperus* extract comprising agathadiol, the compounds agathadiol as such, and the pharmaceutical compositions comprising the same (both the extracts and agathadiol, as disclosed in present invention), may be administered in combination with, prior to, or after administering an inhibitor of one or more enzymes responsible for the degradation of the endocannabinoids, i.e. may be administered in combination with, prior to, or after administering an inhibitor of one or more enzymes responsible for the degradation of anandamide and/or 2-arachidonoylglycerol. Even more preferably, the plant extract comprising agathadiol, more preferably the *Juniperus* extract comprising agathadiol, the compounds agathadiol as such, and the pharmaceutical compositions comprising the same (both the extracts and agathadiol, as disclosed in present invention), may be administered in combination with, prior to, or after administering an inhibitor of fatty acid amide hydrolase (EC 3.5.1.99) or/and an inhibitor of monoacylglycerol lipase (EC 3.1.1.23).

Typical pharmaceutical compositions include the compounds described above herein associated with pharmaceutically acceptable excipients, which may be a carrier or a diluent, as a way of example. Such compositions can be in the form of a capsule, sachet, paper or other container. In making the compositions, conventional techniques for the preparation of pharmaceutical compositions may be used. For example, the compounds disclosed above herein may be mixed with a carrier, or diluted by a carrier, or enclosed within a carrier that may be in the form of an ampoule, capsule, sachet, paper, or other container. When the carrier serves as a diluent, it may be solid, semi-solid, or liquid material that acts as a vehicle, excipient, or medium for the active compound. The compounds described above herein can be adsorbed on a granular solid container for example in a sachet. Some examples of suitable carriers are water, salt solutions, alcohols, polyethylene glycols, polyhydroxyethoxylated castor oil, peanut oil, olive oil, lactose, terra alba, sucrose, cyclodextrin, amylose, magnesium stearate, talc, gelatine, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose, and polyvinylpyrrolidone. Similarly, the carrier or diluent may include any sustained release material known in the art, such as glyceryl monostearate or glyceryl distearate, alone or mixed with a wax. Said compositions may also include wetting agents, emulsifying and suspending agents, preserving agents, sweetening agents or flavouring agents. The compositions of the invention may be formulated so as to provide quick, sustained, or delayed release of the compounds disclosed herein after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the compounds disclosed herein.

One preferred embodiment disclosed herein refers to the route of administration, that may be any route which effectively transports the compound disclosed above herein, to the appropriate or desired site of action, such as oral, nasal, topical, pulmonary, transdermal or parenteral, e. g., rectal, subcutaneous, intravenous, intraurethral, intramuscular, intranasal, ophthalmic solution or an ointment.

For nasal administration, the compositions may contain the compound disclosed above herein, dissolved or suspended in a liquid carrier, in particular an aqueous carrier, for aerosol application. The carrier may contain additives such as solubilizing agents, e.g., propylene glycol, surfactants, absorption enhancers such as lecithin (phosphatidylcholine), or cyclodextrin, or preservatives such as parabens.

To prepare topical compositions of the invention, the compound disclosed above herein, is placed in a dermatological vehicle as is known in the art. The amount of the compound disclosed above herein to be administered and the compound's concentration in the topical formulations depend upon the vehicle, delivery system or device selected, the clinical condition of the patient, the side effects and the stability of the compound in the formulation. Thus, the physician employs the appropriate preparation containing the appropriate concentration of the compound disclosed above herein, and selects the amount of formulation administered, depending upon clinical experience with the patient in question or with similar patients.

For ophthalmic applications, the compound disclosed above herein, is formulated into solutions, suspensions, and ointments appropriate for use in the eye. The concentrations are usually as discussed above herein for local preparations.

For oral administration, either solid or fluid unit dosage forms can be prepared. For preparing solid compositions such as tablets, the compound disclosed above herein, is mixed into formulations with conventional ingredients such as talc, magnesium stearate, di-calcium phosphate, magnesium aluminum silicate, calcium sulphate, starch, lactose, acacia, methylcellulose, and functionally similar materials as pharmaceutical diluents or carriers.

Capsules are prepared by mixing the compound disclosed above herein with an inert pharmaceutical diluent and filling the mixture into a hard gelatine capsule of appropriate size. Soft gelatine capsules are prepared by machine encapsulation of slurry of the compound with an acceptable vegetable oil, light liquid petrolatum or other inert oil. Fluid unit dosage forms for oral administration such as syrups, elixirs and suspensions can be prepared. The water-soluble forms can be dissolved in an aqueous vehicle together with sugar, aromatic flavouring agents and preservatives to form syrup. An elixir is prepared by using a hydroalcoholic (e. g., ethanol) vehicle with suitable sweeteners such as sugar and saccharin, together with an aromatic flavouring agent. Suspensions can be prepared with an aqueous vehicle with the aid of a suspending agent such as acacia, tragacanthin, methylcellulose and the like.

Appropriate compositions of the invention for parenteral use are apparent to the practitioner of ordinary skill, such as the use of suitable injectable solutions or suspensions. The composition, which is sterile, is suitable for various topical or parenteral routes including intra-dermal, intramuscular, intravascular, and subcutaneous.

In addition to the compounds disclosed above herein, the compositions of the invention may include, depending on the composition and mode of delivery desired, pharmaceutically- acceptable, non-toxic carriers or diluents, which include vehicles commonly used to form pharmaceutical compositions for animal or human administration. The diluents are selected so as not to unduly affect the biological activity of the combination.

Examples of such diluents that are especially useful for injectable formulations are water, the various saline, organic or inorganic salt solutions, Ringer's solution, dextrose solution, and Hank's solution. In addition, the pharmaceutical composition or formulation may include additives such as other carriers; adjuvants; or non-toxic, non-therapeutic, non-immunogenic stabilizers and the like.

Furthermore, excipients can be included in the compositions disclosed. Examples include but are not limited to cosolvents, surfactants, oils, humectants, emollients, preservatives, stabilizers and antioxidants. Any pharmacologically acceptable buffer may be used, such as, tris or phosphate buffers. Effective amounts of diluents, additives, and excipients are those amounts that are effective to obtain a pharmaceutically acceptable formulation in terms of solubility, biological activity, etc.

The pharmaceutical compositions comprising the compound disclosed above herein may be incorporated into a microsphere. The compound disclosed above herein can be loaded into albumin microspheres, from which it is possible to recover such microspheres in a dry powder for nasal administration. Other materials suitable for the preparation of microspheres include agar, alginate, chitosan, starch, hydroxyethyl starch, albumin, agarose, dextran, hyaluronic acid, gelatine, collagen, and casein. The microspheres can be produced by various processes known to the person skilled in the art such as a spray drying process or an emulsification process.

For example, albumin microspheres can be prepared by adding rabbit serum albumin in phosphate buffer to olive oil with stirring to produce water in oil emulsion. Glutaraldehyde solution is then added to the emulsion and the emulsion stirred to cross-link the albumin. The microspheres can then be isolated by centrifugation, the oil removed, and the spheres washed, e. g., with petroleum ether followed by ethanol. Finally, the microspheres can be sieved and collected and dried by filtration.

Starch microspheres can be prepared by adding a warm aqueous starch solution, e. g. of potato starch, to a heated solution of polyethylene glycol in water with stirring to form an emulsion. When the two-phase system has formed (with the starch solution as the inner phase) the mixture is then cooled to room temperature under continued stirring whereupon the inner phase is converted into gel particles. These particles are then filtered off at room temperature and slurred in a solvent such as ethanol, after which the particles are again filtered off and laid to dry in air. The microspheres can be hardened by well-known cross-linking procedures such as heat treatment or by using chemical cross-linking agents. Suitable agents include dialdehydes, including glyoxal, malondialdehyde, succinicaldehyde, adipaldehyde, glutaraldehyde and phthalaldehyde, diketones such as butadione, epichlorohydrin, polyphosphate, and borate. Dialdehydes are used to cross-link proteins such as albumin by interaction with amino groups, and diketones form Schiff bases with amino groups. Epichlorohydrin activates compounds with nucleophiles such as amino or hydroxyl to an epoxide derivative.

Another preferred embodiment of the invention is the dosage scheme of the compounds described above herein. The term "unit dosage form" refers to physically discrete units suitable as unitary dosages for subjects, e. g., mammalian subjects, e. g. humans, dogs, cats, and rodents, each unit containing a predetermined quantity of active material calculated to produce the desired pharmaceutical effect in association with the required pharmaceutical diluent, carrier or vehicle. The specifications for the unit dosage forms of this invention are dictated by and dependent on (a) the unique characteristics of the compound disclosed above herein and the particular effect to be achieved and (b) the limitations inherent in the art wherein said compound for use in humans and animals. Examples of unit dosage forms are tablets, capsules, pills, powder packets, wafers, suppositories, granules, cachets, teaspoonfuls, tablespoonfuls, dropperfuls, ampoules, vials, aerosols with metered discharges, segregated multiples of any of the foregoing, and other forms as herein described. The compositions disclosed herein can be included in kits, which can contain one or more-unit dosage forms of the composition and instructions for use to treat one or more of the diseases described herein.

Slow or extended-release delivery systems, including any of a number of biopolymers (biological-based systems), systems employing liposomes, colloids, resins, and other polymeric delivery systems or compartmentalized reservoirs, can be utilized with the compositions described herein to provide a continuous or long-term source of therapeutic compound. Such slow release systems are applicable to formulations for delivery via topical, intraocular, oral, and parenteral routes.

Additionally, the extracts and compounds disclosed in present application may also be used in nutraceutical applications, combined with nutraceutical excipients or integrated in food complements.

In particular, another embodiment of present application refers to a nutraceutical composition comprising a plant extract which comprises agathadiol and at least one nutraceutical or alimentary acceptable excipient, for use in preventing or alleviating the symptoms of a disease or condition responsive to PAM of CB1R.

Yet another embodiment of present invention refers to a nutraceutical composition agathadiol and a nutraceutical or alimentary acceptable excipient, for use in preventing or alleviating the symptoms of a disease or condition responsive to PAM of CB1R.

Another aspect of the invention refers to a method of preventing or alleviating the symptoms of a disease or condition responsive to PAM of CB1R in a subject in need thereof, wherein said method comprises administering to said subject an effective amount of a nutraceutical composition comprising agathadiol and at least one nutraceutical or alimentary acceptable excipient.

Yet another embodiment of present invention refers to a method of preventing or alleviating the symptoms of a disease or condition responsive to PAM of CB1R in a subject in need thereof, wherein said method comprises administering to said subject an effective amount of a nutraceutical composition comprising a plant extract which comprises agathadiol and at least one nutraceutical or alimentary acceptable excipient.

For the purposes of present invention, an effective amount of the compound, when said effective amount is referred to a nutraceutical use of said compound, is that which provides an objective or subjective prevention of the appearance, or relief, of the symptoms of a disease or condition responsive to PAM of CB1R, in a subject, as noted by the subject or other qualified observer, without producing intolerable adverse effects.

More preferably, the plant extract which comprises agathadiol is a *Juniperus* extract, and even more preferably a supercritical CO₂ extract.

In a preferred embodiment the nutraceutical composition is used for preventing or alleviating neuropathic pain.

The nutraceutical compositions disclosed in present invention can also be included in a variety of food preparations, such as dairy products: yoghurt, quark, cheese (such as cottage cheese, cream, processed, soft and hard cheese), fermented milk, powdered milk, a fermented dairy product, ice cream, a product based on fermented cereal, milk-based powder, drinks, and pet food.

The term "food preparations" is used here in its broadest sense, including any type of product, in any presentation form, that can be ingested by a human or an animal, except for pharmaceutical and veterinarian products. Examples of other food preparations are meat products (such as pates, sausages, salami or spreadable meat), spreadable chocolate, fillers (such as truffles, cream) and cake icing, chocolate, sweets (such as caramel, fondant or toffee), baked products (pastries, biscuits), sauces and soups, fruit juices and coffee whitener. Food products of particular interest include food supplements and baby formulas.

The nutraceutical composition according to the invention can also be used as an ingredient in other food products. Consequently, in another aspect of the invention food products are provided that contain the compositions of the invention as well as appropriate amounts of edible ingredients. Preferably, the nutraceutical composition for use according to the invention is a food supplement. For the purpose of the present invention, the term "food supplement" refers to the fraction of the food that is used to complement human or animal food. If the nutraceutical composition for use according to the invention is used as a food supplement, it can be administered as such or can be mixed with a suitable potable liquid, such as water, yoghurt, milk or fruit juice, or can be mixed with solid or liquid food. In this context, the food supplement can be in the form of tablets, pills, capsules, granules, powders, suspensions, sachets, pastilles, sweets, bars, syrups and corresponding forms for administration, generally as unit doses.

### Examples

The examples of the present invention described below aim to illustrate its preferred embodiments without limiting its scope of protection.

### Example 1: isolation of Juniperus communis berries extracts

Juniper fractions were obtained by different solvent extraction-fractionation methods (Acetonic extract, essential oil and ethanolic extract).

### Preparation of an ethanolic extract

Juniper berries (10 g) are powdered and extracted twice with ethanol (1:10 w/V) under stirring for 12 h. The suspension is then filtered and evaporated with rotary evaporator at 45 °C to obtain a black creamy extract (660 mg).

### Isolation of fractions F1-F3:

Fractions F1, F2 and F3 correspond, respectively, to the petroleum ether, ethyl acetate and methanol fractions obtained by an increasing polarity (petroleum ether, ethyl acetate, tetrahydrofuran) solid phase extraction method from a global initial extract prepared by extraction with acetone.

Juniper berries (100 g) were powdered and extracted twice with acetone (1:10 w/V) under stirring for 12 h. The suspension was then filtered and evaporated with rotary evaporator at 45 °C to obtain a black creamy extract (18.8 g). This latter was dissolved in the minimum amount of acetone, adsorbed with silica (1:3 w/w) and evaporated to obtain a powder that stratified on a layer of Celite (1:3 w/w g) packed with petroleum ether in a funnel with a sintered filter. Next petroleum ether, EtOAc and tetrahydrofuran (each solvent is added 1:30 w/V) were sequentially passed through the filter under vacuum. The three filtrates (petroleum ether, ethyl acetate, tetrahydrofuran) were evaporated at rotary evaporator at 45 °C to afford three fractions respectively F1 (3.27 g), F2 (6.17 g) and F3 (1.97 g).

### Isolation of fractions F4 and F5:

F4 and F5 correspond, respectively, to the acidic and neutral phases obtained by extraction liquid/liquid with 2% NaOH from a global initial extract prepared by extraction with acetone. F5.S1-7 fractions correspond to neutral phase (F5) purified on silica gel. F5.S8-12 correspond to neutral phase (F5) purified on silica gel and filtered on alumina.

Juniper berries (300 g) are powdered and extracted with acetone (1:10 w/V) in a percolator for 12 h. The extract is then filtered and evaporated with rotary evaporator at 45 °C to obtain a black creamy extract (48 g). 23 g of the latter are dissolved in 150 mL of EtOAc and washed twice with 100 mL of 2% NaOH water solution (F4). The final organic fraction in then anhydrified with Na2SO4 and completely concentrated affording a yellow oil (F5 11.4 g) which was further purified by gravity CC on silica gel (1:50 w/V) using a gradient of petroleum ether-EtOAc (from 95:05 to 30:70) affording seven fractions (F5.S1-7). All fractions F5.S1-7 had been filtered on alumina finally resulting in F5.S8-12.

### Example 2. Juniper polar fractions enhance CB1R activity on cAMP signalling pathway induced by the agonist CP-55,940.

The determination of CB1R activity was carried out using the HEK293T-CB1-CRE-Luc cells stably transfected with the human CB1R cDNA, and a construction of CRE-Luc that contains as promoter six consensus cAMP responsive elements (CRE) linked to firefly luciferase reporter gene. The increase in cAMP levels activates the CRE-Luc system, inducing the expression of the luciferase reporter gene. Briefly, HEK293T-CB1-CRE-Luc cells were cultured in supplemented DMEM (Sigma) medium containing 10% FBS (Gibco) and 1% antibiotics penicillin/streptomycin (Sigma) (DMEM complete medium) and maintained at 37° C in a humidified atmosphere of 5% CO₂. The day before the assay 10⁴ cells per well were seeded in 96 well-plates and incubated overnight. For determining CB1R positive allosteric modulation (PAM) activity, HEK293T-CB1-CRE-Luc cells were treated with increasing concentrations of the fractions for 30 min and then treated with the CB1R orthosteric agonist CP-55,940 at 2.5 µM. For CB1R agonistic activity, HEK293T-CB1-CRE-Luc cells were treated with increasing concentrations of the fractions. CP-55,940 alone, a CB1R orthosteric agonist, was used at 2.5 µM as positive control of cAMP signalling pathway activated by a CB1R-dependent orthosteric mechanism; and cells without stimulus were used as negative control. In both assays, after 6 h of stimulation, cells were washed twice with PBS IX and lysed in 100 µL lysis buffer containing 25 mM Tris-phosphate (pH 7.8), 8 mM MgCl₂, 1 mM DTT, 1% Triton X-100, and 7% glycerol during 15 min at room temperature in a horizontal shaker. Luciferase activity was measured using a TriStar2 Berthold/LB942 multimode reader (Berthold Technologies) following the instructions of the luciferase assay kit (Promega, Madison, WI, USA). The RLUs (relative light units) were calculated and: (i) For allosteric activity, results were expressed as percentage of CB1R activity normalized to 100% stimulation induced by 2.5 µM CP-55,940, according to the following formula: Percentage of CB1R activity = 100 ^{∗} (T - B) / (C - B); where T are the RLUs of the cells treated with the fractions and 2.5 µM CP-55,940; C are the RLUs of the cells treated with CP-55,940 alone, and B are the RLUs of the cells without stimulus. CB1R activity ≥ 150% was considered CB1R PAM activity (ii) For the CB1R agonistic activity, results were expressed as a fold change over non-stimulated cells according to the following formula: Fold change of CB1R activity = T / B; where T are RLUs of the cells treated with the fractions and B are the RLUs of the cells without stimulus. CB1R activity ≥ 2.5-fold increase is considered CB1R agonistic activity.

Juniper essential oil had not effect on CB1R activity induced by the orthosteric agonist CP-55,940, activity considered the 100% of receptor stimulation. In contrast, the ethanolic global extract showed, from the lowest tested concentration (5 µg/mL), a strong PAM CB1R effect on cAMP signalling induced by the agonist CP-55,940 enhancing the CB1R activity over 250% of activity (Figure 1 and 2A). In the case of the acetonic global extract the effect on CB1R activity was variable depending on the polarity of the resultant fractions.

Regardless the solvent extraction-fractionation method used, the less polar Juniper fractions (F1 and F4) from the global acetonic extract did not show effect on CB1R activity (100% of activity) (Figure 1). However, as the polarity of juniper fractions (F2, F3, F5 and F5.S7) increased, a strong PAM CB1R effect on CP-55,940 induced CB1R activity was observed in a concentration-dependent manner from the lowest tested concentration reaching activity values over 500% in all of them (Figure 1 and Figure 2A). To discard if this the enhancement of CB1R activity was due to an orthosteric CB1R agonistic effect of the fractions, this potential effect was also assayed (Figure 2B).

Data showed that F3 fraction and the ethanolic global extract did not show CB1R agonistic activity (Fold change of CB1R activity remained below 2.5-fold) (Figure 2B). However, although the rest of fractions (F2, F5 and F5.S7) showed a slightly CB1R agonistic activity (Fold change of CB1R receptor activity was slightly superior to 2.5-fold), this effect was residual (Figure 2B).

Thus, it can be concluded that polar Juniper fractions independently of the solvent extraction-fractionation method used exert a clear PAM CB1R activity.

### Example 3. Juniper CO2-se total extract exerts a strong PAM CB1R activity on cAMP signalling induced by the agonist CP-55,940 and induces an orthosteric CB1R agonistic activity on β-arrestin signalling pathway.

Juniper CO2-se total extract and fractions were obtained by a liquid/liquid (petroleum ether:methanol/water 8:2 v/v) partition method from the Juniper CO2-se total extract. F1 CO2-se and F2 CO2-se fractions correspond, respectively, to petroleum ether fraction and to petroleum ether fraction filtered on alumina. F3 CO2-se and F4 CO2-se fractions correspond, respectively, to methanol/water fraction and to methanol/water fraction filtered on alumina.

CB1R PAM activity of Juniper CO2-se total extract was determined by performing a concentration curve on HEK293T-CB1-CRE-Luc in the presence of the CBR1 agonist CP-55,940 (Figure 3 and 4A). Data from this assay showed a clear concentration-dependent effect of the Juniper CO2-se total extract, being the CB1R PAM effect on cAMP signalling pathway very strong at the highest tested concentration (CB1R activity > 700%) (Figure 4A). To confirm this effect, the orthosteric CB1R agonistic activity was evaluated in the same cell line. These data showed a very weak CB1R agonistic activity of the extract (Fold change of CB1R receptor activity was slightly superior to 2.5-fold) (Figure 4B). Indeed, this orthosteric effect remained constant regardless the concentration, suggesting that Juniper CO2-se total extract exerts CB1R PAM activity (Figure 4A and 4B).

To confirm this CB1R PAM behaviour, CB1R biased signalling assays were performed. The determination of CB1R-mediated signalling pathways activity was carried out using the CB1 MTXNomad HEK293 cell line (Innoprot, Derio, Spain), stably transfected with the human CB1R cDNA and two Nomad Biosensors (Green Fluorescent β-arrestin Nomad Biosensor and Red Fluorescent cAMP Nomad Biosensor) designed for high throughput screening analysis of the CB1R response that results in a cellular cAMP and β-arrestin signalling pathway regulation. The activation of these signalling pathways mediated by CB1R leads to a change in the cellular localization of the Nomad biosensors and to an increment in the intensity of the biosensor's fluorescent signals. Briefly, 3x10⁴ cells per well were seeded in black clear-bottom 96-well plates diluted in 200 µL of Opti-MEM medium (Ref. #51985034. Life Technologies, Carlsbad, CA, USA) and cultured overnight at 37° C in a humidified atmosphere of 5% CO₂. Then, the cells were treated with increasing concentrations of the Juniper CO2-se total extract. CP-55,940, a CB1R agonist, was used at 2.5 µM alone as positive control of cAMP and β-arrestin signalling pathway activated by a CB1R-dependent mechanism and cells without stimulus were used as negative control. After 24 hours of stimulation, the medium was removed and replaced by 100 µL of Dulbecco's Phosphate Buffered Saline with MgCl₂ and CaCl₂ (Ref. #D8662. Sigma-Aldrich. St. Louis, MO, USA). Then, the plate was read using the IncuCyte^{™} Live-Cell Imaging Systems (Essen BioScience) at the appropriate filters. For cAMP Nomad biosensor fluorescent signal (green) the excitation and emission peaks were 592 nm and 650 nm, respectively; and for β-Arrestin Nomad biosensor fluorescent signal (red) the excitation and emission peaks were 482 nm and 502 nm, respectively. Data were analysed by the total green and red fluorescence object integrated intensity (GCU x µm² x Well) using the IncuCyte FLR software. The obtained green and red fluorescence intensity units were expressed as fold change of cAMP and β-arrestin signalling pathway activation, respectively, calculated according to the following formula: Fold change of cAMP and β-arrestin signalling pathway activation = T / B, where T are green or red fluorescence intensity units of the cells treated with the fractions or compounds and B are fluorescence intensity units of the cells without stimulus. Activity ≥ 2.5-fold is considered CB1R agonistic activity for the specific signalling pathway assayed.

This assay confirmed the CB1R PAM activity of Juniper CO2-se total extract on cAMP signalling pathway (Figure 5A). Equal as in HEK293T-CB1-CRE-Luc assay which measured cAMP (Figure 4B), the CB1 Nomad biosensor for cAMP showed a very weak CB1R agonistic activity of the extract which remained constant regardless the concentration (Fold change not superior to 3.5-fold), confirming the previously obtained CB1R PAM results (Figure 4B and 5A). In contrast, the obtained data for β-Arrestin signalling showed a strong CB1R agonistic activity reaching values if activity over 6-fold (Figure 5B). Thus, it can be concluded that Juniper CO2-se total extract exerts CB1R PAM activity for cAMP signalling pathway and an orthosteric CB1R agonistic effect on β-Arrestin signalling pathway, which revealed the CB 1R biased agonistic behaviour of the extract.

### Example 4. Polar Juniper derived fractions from CO2-se total extract maintain the CB1R PAM activity on cAMP signalling induced by the agonist CP-55,940.

Juniper fractions obtained by a liquid/liquid (petroleum ether: methanol/water 8:2 v/v) partition method from the Juniper CO2-se total extract were evaluated in CB1R PAM activity (Figure 3 and 6A). As in example 2, the less polar Juniper fractions (F1 CO2-se and F2 CO2-se) did not show effect on CB1R activity (∼100% of activity) (Figure 3). However, as increasing polarity of juniper fractions (F3 CO2-se and F4 CO2-se) a strong concentration-dependent CB1R PAM effect was observed from the lowest tested concentrations, and mainly, in the fraction F3 CO2-se which reached an activity value over 700% at the maximum evaluated concentration (Figure 3 and Figure 6A). To discard if this the increased of CB1R activity was due to an orthosteric CB1R agonistic activity of the fractions, this effect was assayed (Figure 6B). Data showed that F3 CO2-se fraction exerts a strong CB1R agonistic activity on cAMP signalling pathway (Activity superior to 6-fold) (Figure 6B). In contrast, F4 CO2-se fraction, which derived from the previous one after an additional filtered-step on alumina, showed a residual CB1R agonistic activity cAMP signalling pathway, confirming the CB1R PAM activity of the fraction (Figure 6B). Thus, it can be concluded that the filtration on alumina of the polar F3 CO2-se Juniper fractions removed compounds that exert orthosteric CB1R agonistic activity, and the CB1R PAM activity remains intact.

### Example 5. Agathadiol isolated from F4 CO2-se Juniper fraction exerts a strong CB1R PAM activity on cAMP signalling induced by the agonist CP-55,940.

After the analysis of the F4 CO2-se fraction phytochemical profiling 10 compounds were identified and isolated: agathadic acid, yatein, agathadiol, beta-farnese, beta-elemolo, germacrene D, β-myrcene, sabinene and α-pinene. Pure compounds were evaluated in CB1R activity and only agathadiol showed effect on CB1R (Figure 7A).

For the isolation of agathadiol a sample of carbonic extract (30.52 g - Flavex^{®} *Juniperus communis* carbonic extract) was dissolved in 300 mL of MeOH/Water 90:10 and dewaxed washing with petroleum ether (30 mL x 3). After the defatting step, the hydro alcoholic fraction is concentrated at rotary evaporator (45 °C) to recover an aqueous mixture. This latter is diluted with brine (20 mL) and extracted twice with DCM (20 mL x 2), anhydrified with Na₂SO₄ and completely concentrated affording a yellow oil (4.90 g) which was filtered on neutral alumina (10:30 w/w) with DCM to remove acid compounds. This final fraction (4.53 g) was further purified by gravity CC on silica gel (1:50 w/V) using a gradient of petroleum ether-EtOAc (from 90:10 to 30:70) affording five fractions (I-V). The polar one, fraction V (179 mg), was finally purified with HPLC (Hichrom, 250x25 mm, silica, Jasco) and petroleum ether-EtOAc gradient (0-10 min: 60:40, 10-30 min: 40:60, 30-40 min: 40:60) to recover Agathadiol (13 mg).

Agathadiol showed a very strong effect on CB1R activity enhancing the CB1R activity induced by CP-55,940 to values over 1000% of activity (Figure 7A). To assess if the increased of CB1R activity was due to an orthosteric CB1R agonistic activity of agathadiol, this effect was assayed (Figure 7B). Data showed that agathadiol exerts a medium CB1R agonistic activity on cAMP signalling pathway at high concentrations (Figure 7B). However, this activity appears residual at the lower tested concentration were the agathadiol showed the strongest CB1R PAM activity (Figure 7B). Thus, it can be concluded that agathadiol exerts clear CB1R PAM activities at a range of concentrations.

## Claims

1. A plant extract comprising agathadiol for use in the prevention and/or treatment of a disease or condition responsive to positive allosteric modulators of cannabinoid type 1 receptor

2. The plant extract for use according to claim 1, wherein said extract is a *Juniperus communis* extract.

3. The plant extract for use according to any of claims 1 or 2, wherein the disease or condition responsive to allosteric modulators of cannabinoid type 1 receptor is selected from the group consisting of neurological diseases, psychiatric diseases, glaucoma, inflammation, metabolic and eating disorders, pain, additive behaviour and substance-use disorders.

4. Agathadiol for use in the prevention and/or treatment of a disease or condition responsive to positive allosteric modulators of cannabinoid type 1 receptor.

5. Agathadiol for use according to claim 4, wherein the disease or condition responsive to allosteric modulators of cannabinoid type 1 receptor is selected from the group consisting of neurological diseases, psychiatric diseases, glaucoma, inflammation, metabolic and eating disorders, pain, additive behaviour and substance-use disorders.

6. A pharmaceutical composition comprising a plant extract which comprises agathadiol and at least one pharmaceutically acceptable excipient, for use in the prevention and/or treatment of a disease or condition responsive to positive allosteric modulators of cannabinoid type 1 receptor.

7. The pharmaceutical composition for use according to claim 6, wherein said plant extract is a *Juniperus communis* extract.

8. A pharmaceutical composition comprising agathadiol and at least one pharmaceutically acceptable excipient, for use in the prevention and/or treatment of a disease or condition responsive to positive allosteric modulators of cannabinoid type 1 receptor.

9. The pharmaceutical composition for use according to any of claims 6 to 8, wherein the disease or condition responsive to allosteric modulators of cannabinoid type 1 receptor is selected from the group consisting of neurological diseases, psychiatric diseases, glaucoma, inflammation, metabolic and eating disorders, pain, additive behaviour and substance-use disorders.

10. The pharmaceutical composition for use according to any of claims 6 to 9, wherein said pharmaceutical composition is administered in combination with, prior to, or after an inhibitor of one or more enzymes responsible for the degradation of an endogenous orthosteric ligand of cannabinoid type 1 receptor.

11. The pharmaceutical composition for use according to any of claims 6 to 10, wherein said pharmaceutical composition is administered in combination with, prior to, or after an inhibitor of one or more enzymes responsible for the degradation of anandamide or 2-arachidonoyl-glycerol.

12. A nutraceutical composition comprising a plant extract which comprises agathadiol, and at least one nutraceutical or food acceptable excipient, for use in preventing or alleviating the symptoms of a disease or condition responsive to positive allosteric modulators of cannabinoid type 1 receptor.

13. The nutraceutical composition for use according to claim 12, wherein the plant extract is a *Juniperus communis* plant extract.

14. A nutraceutical composition comprising agathadiol and at least one nutraceutical or food acceptable excipient, for use in preventing or alleviating the symptoms of a disease or condition responsive to positive allosteric modulators of cannabinoid type 1 receptor.

15. The nutraceutical composition for use according to claim 12 to 14, wherein the disease or condition responsive to positive allosteric modulators of cannabinoid type 1 receptor is selected from the group consisting of neurological diseases, psychiatric diseases, glaucoma, inflammation, metabolic and eating disorders, pain, additive behaviour and substance-use disorders.
